# EUROPEAN PATENT APPLICATION

(11) **EP 4 663 756 A1**
(43) Date of publication of application: **17.12.2025**
(21) Application number: 24767424.5
(22) Date of filing: 07.03.2024
(51) Int. Cl.: C12N 9/88, C12P 7/625, C12N 15/70

(54) **5'UTR VARIANT SEQUENCE OF GENE ENCODING PHOSPHOENOLPYRUVATE CARBOXYLASE, AND USES THEREOF**

(30) Priority: 07.03.2023 KR 20230030157
(71) Applicant: CJ CheilJedang Corporation, Seoul 04560 (KR)
(72) Inventor: SEOK, Jooyeon, Seoul 04560 (KR); CHOI, Su Jin, Seoul 04560 (KR); HONG, Eunsoo, Seoul 04560 (KR); LEE, Ji Sun, Seoul 04560 (KR)
(74) Representative: Meissner Bolte Partnerschaft mbB
(86) International application number: PCT/KR2024/002917
(87) International publication number: WO 2024/186136

(57) **Abstract**

The present application relates to: a variant gene encoding phosphoenolpyruvate carboxylase, which comprises a variant 5'-untranslated region (5'UTR), the variant gene comprising a ribosome-binding site (RBS) variant sequence; a polyhydroxyalkanoate (PHA)-producing microorganism comprising the gene; and a PHA production method using same.

## Description

### [Technical Field]

The present disclosure relates to a mutant gene encoding phosphoenolpyruvate carboxylase including a mutant 5'-untranslated region (5'UTR), a polyhydroxyalkanoate (PHA)-producing microorganism including the gene, and a method of producing PHA using the microorganism.

### [Background Art]

As the problems of environmental pollution have emerged due to the accumulation of petroleum-based recalcitrant plastics, biodegradable bioplastics to replace the same are receiving attention. Polyhydroxyalkanoate (PHA), which is one of bioplastic materials, is a polyester-based natural polymer accumulated in microorganisms. PHA is a biodegradable material, does not generate toxic waste, and is known as an eco-friendly polymer with thermoplastic and elastic properties, etc. Among PHA, the most representative known material is poly-3-hydroxybutyrate (P3HB) which is polymerized using a 3-hydroxybutyrate (3HB) monomer. P3HB has physical properties similar to those of polypropylene which is a synthetic resin used in automobile parts, home appliance parts, etc. Since P3HB can be relatively easily synthesized by microorganisms, many studies on the production thereof have been commercially conducted. However, there are limitations that P3HB has brittleness due to high crystallinity and is difficult to process because it decomposes near its melting point. Therefore, studies have been conducted to produce a PHA copolymer through blending with new materials or copolymerization of monomers. Among them, a poly(3-hydroxybutyrate-co-4-hydroxybutyrate (P(3HB-co-4HB)) copolymer is a material having characteristics as an elastic body due to the addition of 4-hydroxybutyrate (4HB) monomer. The physical properties of PHA change depending on the addition ratio of the 4HB monomer, and PHA may be used in the production of various products. Therefore, it is attracting attention as a promising biodegradable polymer material.

### [Disclosure]

### [Technical Problem]

The present inventors found that a 5'-untranslated region (5'UTR) mutant sequence of the present disclosure can be used to control the carbon flux of the TCA cycle, in particular, to control the 4-hydroxybutyrate (4HB) content of polyhydroxyalkanoate (PHA)-producing microorganisms, thereby providing PHA having desired physical properties, and completed the present the present disclosure.

### [Technical Solution]

An object of the present disclosure is to provide a mutant polynucleotide encoding a mutant 5'-untranslated region (5'UTR) including a sequence in which any one or more bases selected from the group consisting of bases corresponding to positions 3, 4, 5, 7, 8, and 10 in a nucleotide sequence of SEQ ID NO: 1 are mutated.

Another object of the present disclosure is to provide a mutant polynucleotide including a nucleotide sequence encoding phosphoenolpyruvate carboxylase, the mutant polynucleotide further including a nucleotide sequence encoding a mutant 5'UTR including a sequence in which any one or more bases selected from the group consisting of bases corresponding to positions 3, 4, 5, 7, 8, and 10 in a nucleotide sequence of SEQ ID NO: 1 are mutated.

Still another object of the present disclosure is to provide a polyhydroxyalkanoate-producing microorganism, the microorganism comprising a mutant polynucleotide encoding a mutant 5'UTR in which any one or more bases selected from the group consisting of bases corresponding to positions 3, 4, 5, 7, 8, and 10 in a nucleotide sequence of SEQ ID NO: 1 are mutated; or a mutant polynucleotide including a nucleotide sequence encoding phosphoenolpyruvate carboxylase, the mutant polynucleotide further including the nucleotide sequence encoding the mutant 5'UTR.

Still another object of the present disclosure is to provide a method of producing polyhydroxyalkanoate, the method including the step of culturing, in a medium, a polyhydroxyalkanoate-producing microorganism , the microorganism including a mutant polynucleotide encoding a mutant 5'UTR in which any one or more bases selected from the group consisting of bases corresponding to positions 3, 4, 5, 7, 8, and 10 in a nucleotide sequence of SEQ ID NO: 1 are mutated; or a mutant polynucleotide including a nucleotide sequence encoding phosphoenolpyruvate carboxylase, the mutant polynucleotide further including the nucleotide sequence encoding the mutant 5'UTR.

### [Advantageous Effects]

A 5'-untranslated region (5'UTR) mutant sequence of the present disclosure may be used to control the carbon flux of the TCA cycle, in particular, to control the 4-hydroxybutyrate (4HB) content of polyhydroxyalkanoate (PHA)-producing microorganisms, thereby providing PHA having desired physical properties.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, those skilled in the art will recognize, or be able to ascertain using no more than routine experimentation, many equivalents to the specific embodiments of the disclosure described herein. Further, these equivalents should be interpreted to fall within the present disclosure.

One aspect of the present disclosure provides a mutant polynucleotide encoding a mutant 5'-untranslated region (5'UTR) including a sequence in which any one or more bases selected from the group consisting of bases corresponding to positions 3, 4, 5, 7, 8, and 10 in a nucleotide sequence of SEQ ID NO: 1 are mutated.

As used herein, the term "5'-untranslated region (5'UTR)" refers to a region that exists at the 5'-end of an mRNA transcript, but is not translated into amino acids. The translation of mRNA into a protein begins with the binding of 30S ribosomal subunit to the 5'UTR. Specifically, when 16S rRNA (16S ribosomal RNA) in the 30S ribosomal subunit binds to RBS within the 5'UTR, and tRNA recognizes and binds to the start codon (AUG) of mRNA, translation into proteins begins. It is known that the ribosome binding site of the 5'UTR and the start codon exist approximately 6 to 8 nucleotides away.

In the present disclosure, the 5'UTR includes a sequence, in which a sequence complementary to the sequence of the 3'-end of 16S rRNA, i.e., the Shine-Dalgarno sequence is conserved and a sequence before and after the Shine-Dalgarno sequence is modified.

The "5'UTR sequence modified" or "modified 5'UTR" means that one or more bases in the nucleotide sequence constituting the 5'UTR are modified, and such modification may include substitutions, additions, deletions, or inversions of bases. For a representative example, the 5'UTR may include a sequence, in which the Shine-Dalgarno sequence is conserved and one or more bases in the nucleotide sequence existing before and after the Shine-Dalgarno sequence are modified by substitution, addition, deletion, or inversion.

The 5'UTR may include a ribosome binding site (RBS).

As used herein, the term "ribosome binding site (RBS)" refers to a region (structure) present inside an mRNA chain, to which ribosomes directly bind, and which is able to initiate translation, or a region (structure) of a DNA chain that causes the region to be transcribed. Most prokaryotes possess RBS which is a short sequence, which allows the ribosome to easily recognize and bind to mRNA, near the 5' upstream from a start codon (usually 'AUG') on mRNA containing an open reading frame (ORF). RBS includes a sequence complementary to the sequence of the 3'-end of the 16S rRNA, which is referred to as the Shine-Dalgarno sequence.

The mutant 5'UTR of the present disclosure may be a mutant 5'UTR, in which one or more bases in the nucleotide sequence of SEQ ID NO: 1 of the wild-type 5'UTR are mutated.

In the present disclosure, the nucleotide sequence of SEQ ID NO: 1 may be a 5'-untranslated region (5'UTR) of a gene encoding phosphoenolpyruvate carboxylase. The 5'UTR of the present disclosure may include, may have, may consist of, or may essentially consist of the nucleotide sequence of SEQ ID NO: 1.

The nucleotide sequence of SEQ ID NO: 1 may include a nucleotide sequence having at least 70%, 75%, 76%, 80%, 84%, 85%, 88%, 90%, 92%, 95%, 96%, 97%, 98%, 99%, 99.5%, 99.7%, or 99.9% or more homology or identity to the nucleotide sequence described by SEQ ID NO: 1. Further, it is apparent that any nucleotide sequence with deletion, modification, substitution, conservative substitution or addition of some sequence may also be included within the scope of the present disclosure, as long as the nucleotide sequence has such a homology or identity and exhibits efficacy corresponding to the 5'UTR.

In one embodiment, the 5'UTR of the present disclosure is a nucleotide sequence including the nucleotide sequence of SEQ ID NO: 1, and may include a nucleotide sequence of SEQ ID NO: 19.

In one embodiment, the 5'UTR of the present disclosure, in which one or more bases are mutated, may be used interchangeably with a "mutant RBS", a "mutant RBS-containing sequence", or a "mutant 5'UTR", in which the RBS sequence in the 5'UTR is mutated.

More specifically, the mutant 5'UTR of the present disclosure may have mutations in any one or more bases of the bases corresponding to positions 3 to 10 from the 5' terminus in the nucleotide sequence of SEQ ID NO: 1.

Much more specifically, the mutant 5'UTR of the present disclosure may include a polynucleotide in which any one or more bases selected from the group consisting of the bases corresponding to positions 3, 4, 5, 7, 8, and 10 in the nucleotide sequence of SEQ ID NO: 1 are mutated. Examples thereof may include a polynucleotide in which any one, two or more, three or more, four or more, five or more, or six or more selected from the bases corresponding to the above-described positions are mutated.

For example, the mutations of the present disclosure may refer to substitution of specific bases with bases different from the bases before the substitution. In other words, the mutant 5'UTR of the present disclosure may include a polynucleotide in which any one or more bases selected from the group consisting of the bases corresponding to positions 3, 4, 5, 7, 8, and 10 in the nucleotide sequence of SEQ ID NO: 1 are substituted with different bases.

Specifically, the mutant 5'UTR of the present disclosure may include a polynucleotide in which T which is the base corresponding to position 3 in the nucleotide sequence of SEQ ID NO: 1 is substituted with a base selected from the group consisting of G, C, and A. The mutant 5'UTR of the present disclosure may include a polynucleotide in which C which is the base corresponding to position 4 in the nucleotide sequence of SEQ ID NO: 1 is substituted with a base selected from the group consisting of A, G, and T. The mutant 5'UTR of the present disclosure may include a polynucleotide in which T which is the base corresponding to position 5 in the nucleotide sequence of SEQ ID NO: 1 is substituted with a base selected from the group consisting of G, C, and A. The mutant 5'UTR of the present disclosure may include a polynucleotide in which G which is the base corresponding to position 7 in the nucleotide sequence of SEQ ID NO: 1 is substituted with a base selected from the group consisting of A, C, and T. The mutant 5'UTR of the present disclosure may include a polynucleotide in which G which is the base corresponding to position 8 in the nucleotide sequence of SEQ ID NO: 1 is substituted with a base selected from the group consisting of C, A, and T. Further, the mutant 5'UTR of the present disclosure may include a polynucleotide in which T which is the base corresponding to position 10 in the nucleotide sequence of SEQ ID NO: 1 is substituted with a base selected from the group consisting of G, C, and A. The mutant 5'UTR of the present disclosure may include a polynucleotide including any one or a combination of two or more, three or more, four or more, five or more, or six or more selected from the above-described substitutions.

More specifically, the mutation of the present disclosure may be any one or more selected from the following substitutions:
i) substitution of T with G;
ii) substitution of C with A;
iii) substitution of G with A; and
iv) substitution of G with C.

In one embodiment, the mutant 5'UTR of the present disclosure may include a polynucleotide in which T which is the base corresponding to position 3 in the nucleotide sequence of SEQ ID NO: 1 is substituted with G. The mutant 5'UTR of the present disclosure may include a polynucleotide in which C which is the base corresponding to position 4 in the nucleotide sequence of SEQ ID NO: 1 is substituted with A. The mutant 5'UTR of the present disclosure may include a polynucleotide in which T which is the base corresponding to position 5 in the nucleotide sequence of SEQ ID NO: 1 is substituted with G. The mutant 5'UTR of the present disclosure may include a polynucleotide in which G which is the base corresponding to position 7 in the nucleotide sequence of SEQ ID NO: 1 is substituted with A. The mutant 5'UTR of the present disclosure may include a polynucleotide in which G which is the base corresponding to position 8 in the nucleotide sequence of SEQ ID NO: 1 is substituted with C. Further, the mutant 5'UTR of the present disclosure may include a polynucleotide in which T which is the base corresponding to position 10 in the nucleotide sequence of SEQ ID NO: 1 is substituted with G. The mutant 5'UTR of the present disclosure may include a polynucleotide including any one or a combination of two or more, three or more, four or more, five or more, or six or more selected from the above-described substitutions.

As used herein, the term "corresponding position" refers to bases (nucleotides) at positions listed in a nucleotide sequence or polynucleotide, or bases that are similar, identical, or homologous to those listed in the nucleotide sequence or polynucleotide. As used herein, "corresponding region" generally refers to a similar or corresponding position in a related polynucleotide or a reference polynucleotide.

In the present disclosure, specific numbering may be used for base positions in the nucleotide sequence or polynucleotide used in the present disclosure. For example, by aligning a target nucleotide sequence to be compared with the nucleotide sequence of the present disclosure, it is possible to renumber the position corresponding to the base position of the polynucleotide of the present disclosure.

Much more specifically, the polynucleotide encoding the mutant 5'UTR of the present disclosure may include a nucleotide sequence described by any one selected from SEQ ID NO: 2 to SEQ ID NO: 18.

Further, the polynucleotide encoding the mutant 5'UTR of the present disclosure may have or may include a nucleotide sequence having 70% or more, 75% or more, 76% or more, 80% or more, 84% or more, 85% or more, 88% or more, 90% or more, 92% or more, 95% or more, 96% or more, 97% or more, 98% or more, 99% or more, 99.5% or more, 99.7% or more, 99.9% or more, and less than 100% homology or identity to the entirety of any one sequence selected from SEQ ID NO: 2 to SEQ ID NO: 18, provided that respective mutated sequences in SEQ ID NO: 2 to SEQ ID NO: 18, as compared to the sequence of SEQ ID NO: 1 which is a part of the polynucleotide encoding the wild-type 5'UTR, are fixed, or it may include, may have, may consist of, or may essentially consist of a nucleotide sequence having 70% or more, 75% or more, 76% or more, 80% or more, 84% or more, 85% or more, 88% or more, 90% or more, 92% or more, 95% or more, 96% or more, 97% or more, 98% or more, 99% or more, 99.5% or more, 99.7% or more, 99.9% or more, and less than 100% homology or identity to any one sequence selected from SEQ ID NO: 2 to SEQ ID NO: 18, but is not limited thereto.

Further, it is apparent that any mutant 5'UTR with deletion, modification, substitution, conservative substitution or addition of some sequence may also be included within the scope of the present disclosure, as long as the nucleotide sequence has such a homology or identity and exhibits efficacy corresponding to the mutant 5'UTR of the present disclosure.

In one embodiment, the polynucleotide encoding the mutant 5'UTR of the present disclosure may be a nucleotide sequence including a nucleotide sequence described by any one selected from SEQ ID NO: 2 to SEQ ID NO: 18, and may include a nucleotide sequence described by any one selected from SEQ ID NO: 20 to SEQ ID NO: 36.

As used herein, the term "polynucleotide", which is a polymer of nucleotides, in which nucleotide monomers are linked in a long chain shape by covalent bonds, refers to a DNA or RNA strand having a predetermined or longer length.

As used herein, the term "mutant" refers to a polynucleotide which has a nucleotide sequence different from the nucleotide sequence before modification due to modification of one or more bases, but maintains the functions or properties. Such a mutant may be generally identified by modifying one or more bases of the nucleotide sequence of the polynucleotide and evaluating the properties of the modified polynucleotide. In other words, the ability of the mutant polynucleotide may be increased, unchanged, or decreased, as compared to that of the polynucleotide before being modified. The mutant polynucleotide may be used interchangeably with terms such as variant, modification, modified polynucleotide, modified gene, mutant, mutein, divergent, etc., and is not limited thereto as long as it is a term used with the meaning of variation.

The mutant polynucleotide may be conjugated with other sequences or linkers so as to be identified, purified, or synthesized.

As used herein, the term "corresponding to" refers to bases at positions listed in the polynucleotide, or bases that are similar, identical, or homologous to those listed in the polynucleotide. Identifying the base at the corresponding position may be determining a specific base in a sequence that refers to a specific sequence. As used herein, "corresponding region" generally refers to a similar or corresponding position in a related polynucleotide sequence or a reference polynucleotide sequence.

For example, an arbitrary nucleotide sequence is aligned with SEQ ID NO: 1, and based on this, each base of the nucleotide sequence may be numbered with reference to the base of SEQ ID NO: 1 and the numerical position of the corresponding base. For example, a sequence alignment algorithm as described in the present disclosure may determine the position of a base or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000, Trends Genet. 16:276-277), and the like may be used, but are not limited thereto, and a sequence alignment program, a pairwise sequence comparison algorithm, and the like known in the art may be appropriately used.

As used herein, the term 'homology' or 'identity' means the degree of similarity between two given amino acid sequences or nucleotide sequences and may be expressed as a percentage. The terms 'homology and identity' may often be used interchangeably.

The sequence homology or identity of a conserved polynucleotide or polypeptide is determined by standard alignment algorithms, and the default gap penalty established by the used program may be used together. Substantially, homologous or identical sequences are generally capable of being hybridized with the entirety or a part of the sequence under moderately or highly stringent conditions. It is apparent that hybridization also includes hybridization with a polynucleotide including a general codon or a codon in consideration of codon degeneracy.

Whether any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, the homology, similarity, or identity may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ET AL.](1988) SIAM J Applied Math 48: 1073). For example, BLAST of the National Center for Biotechnology Information, or ClustalW may be used to determine the homology, similarity, or identity.

The homology, similarity, or identity of polynucleotides or polypeptides may be determined by comparing sequence information using, for example, a GAP computer program such as Needleman et al. (1970), J Mol Biol. 48:443 as announced in, for example, Smith and Waterman, Adv. Appl. Math (1981) 2:482. In summary, the GAP program may be defined as the value acquired by dividing the number of similarly aligned symbols (namely, nucleotides or amino acids) by the total number of symbols in the shorter of two sequences. The default parameters for the GAP program may include (1) a binary comparison matrix (including values of 1 for identity and 0 for non-identity) and a weighted comparison matrix of Gribskov et al., (1986) Nucl. Acids Res. 14:6745 (or EDNAFULL (EMBOSS version of NCBI NUC4.4) substitution matrix), as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

In the polynucleotide of the present disclosure, various modifications may be made in the coding region as long as the nucleotide sequence of the mutant gene including the polynucleotide encoding the mutant 5'UTR of the present disclosure is not changed in consideration of codon degeneracy or codons preferred in organisms that are intended to express the mutant gene including the polynucleotide encoding the mutant 5'UTR of the present disclosure.

Further, the polynucleotide of the present disclosure may include a probe that may be prepared from a known gene sequence, for example, a sequence without limitation as long as it is a sequence that can hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al.,Molecular Cloning, A Laboratory Manual, 2nd Edition, Cold Spring Harbor Laboratory press, Cold Spring Harbor, New York, 1989; F.M. Ausubel et al.,Current Protocols in Molecular Biology, John Wiley & Sons, Inc., New York, 9.50-9.51, 11.7-11.8). Examples thereof include conditions under which polynucleotides having higher homology or identity, namely polynucleotides having 70% or more, 75% or more, 76% or more, 80% or more, 84% or more, 85% or more, 88% or more, 90% or more, 92% or more, 95% or more, 96% or more, 97% or more, 98% or more, 99% or more, 99.5% or more, 99.7% or more, 99.8% or more, or 99.9% or more homology or identity are hybridized with each other while polynucleotides having lower homology or identity are not hybridized with each other, or conditions under which washing is performed once, specifically twice to three times at a salt concentration and temperature equivalent to 60°C, 1X SSC, 0.1% SDS, specifically 60°C, 0.1X SSC, 0.1% SDS, more specifically 68°C, 0.1X SSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also include substantially similar nucleic acid sequences as well as isolated nucleic acid fragments that are complementary to the entire sequence.

Specifically, a polynucleotide having homology or identity to the polynucleotide of the present disclosure may be detected using a hybridization condition including a hybridization step at a Tm value of 55°C and the above-described conditions. The Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (for example, J. Sambrook et al., supra).

The mutant polynucleotide encoding the mutant 5'UTR of the present disclosure may regulate translation of a polynucleotide encoding a target protein operably linked thereto into a protein.

For example, the target protein may be phosphoenolpyruvate carboxylase, but is not limited thereto, and the mutant polynucleotide encoding the mutant 5'UTR of the present disclosure may be used universally for the purpose of regulating the translation of polynucleotides encoding various target proteins into proteins.

Another aspect of the present disclosure provides a mutant polynucleotide including a nucleotide sequence encoding phosphoenolpyruvate carboxylase, the mutant polynucleotide further including the nucleotide sequence encoding the mutant 5'UTR including a sequence in which any one or more bases selected from the group consisting of bases corresponding to positions 3, 4, 5, 7, 8, and 10 in a nucleotide sequence of SEQ ID NO: 1 are mutated.

The mutant polynucleotide, the nucleotide sequence of SEQ ID NO: 1, and the mutation and mutant 5'UTR are as described above.

The mutation of the present disclosure may be any one or more selected from the following substitutions:
i) substitution of T with G;
ii) substitution of C with A;
iii) substitution of G with A; and
iv) substitution of G with C.

In one embodiment, the mutant 5'UTR of the present disclosure may include a polynucleotide in which T which is the base corresponding to position 3 in the nucleotide sequence of SEQ ID NO: 1 is substituted with G. The mutant 5'UTR of the present disclosure may include a polynucleotide in which C which is the base corresponding to position 4 in the nucleotide sequence of SEQ ID NO: 1 is substituted with A. The mutant 5'UTR of the present disclosure may include a polynucleotide in which T which is the base corresponding to position 5 in the nucleotide sequence of SEQ ID NO: 1 is substituted with G. The mutant 5'UTR of the present disclosure may include a polynucleotide in which G which is the base corresponding to position 7 in the nucleotide sequence of SEQ ID NO: 1 is substituted with A. The mutant 5'UTR of the present disclosure may include a polynucleotide in which G which is the base corresponding to position 8 in the nucleotide sequence of SEQ ID NO: 1 is substituted with C. Further, the mutant 5'UTR of the present disclosure may include a polynucleotide in which T which is the base corresponding to position 10 in the nucleotide sequence of SEQ ID NO: 1 is substituted with G. The mutant 5'UTR of the present disclosure may include a polynucleotide including any one or a combination of two or more, three or more, four or more, five or more, or six or more selected from the above-described substitutions. The description thereof is as above.

As used herein, the term "phosphoenolpyruvate carboxylase" is an enzyme that promotes the addition of bicarbonate to phosphorenolpyruvate to form oxaloacetate which is a 4-carbon compound and inorganic phosphate, and has a major effect on the carbon flux into the TCA cycle, and the expression level of *ppc* gene encoding the same may affect the 4-hydroxybutyrate (4HB) content of polyhydroxyalkanoate (PHA) produced from microorganisms.

An amino acid sequence of the phosphoenolpyruvate carboxylase may be obtained from NCBI's Genebank which is a known database, etc.

For example, the phosphoenolpyruvate carboxylase of the present disclosure may be derived from microorganisms, but is not limited thereto.

In the present disclosure, the phosphoenolpyruvate carboxylase may include, may have, or may consist of an amino acid sequence described by SEQ ID NO: 50, or may essentially consist of the amino acid sequence.

In the present disclosure, the amino acid sequence of SEQ ID NO: 50 may include an amino acid sequence having at least 70%, 75%, 80% 85%, 90%, 95%, 96%, 97%, 98%, 99%, 99.5%, 99.7% or 99.9% or more homology or identity thereto. Further, it is apparent that any protein having an amino acid sequence with deletion, modification, substitution, conservative substitution or addition of some sequence may also be included within the scope of the present disclosure, as long as the amino acid sequence has such a homology or identity and exhibits efficacy corresponding to that of the protein including the amino acid sequence of SEQ ID NO: 50.

Examples thereof include those having sequence addition or deletion that does not alter the function of the protein of the present disclosure at the N-terminus, C-terminus of the amino acid sequence, and/or inside the amino acid sequence, naturally occurring mutation, silent mutation, or conservative substitution.

The "conservative substitution" means the substitution of one amino acid with another amino acid having similar structural and/or chemical properties. Such an amino acid substitution may generally occur based on similarity in the polarity, charge, solubility, hydrophobicity, hydrophilicity, and/or amphipathic nature of residues. Usually, conservative substitution may hardly affect or not affect the activity of the proteins or polypeptides.

In the present disclosure, a gene encoding the phosphoenolpyruvate carboxylase may be a *ppc* gene.

A nucleotide sequence of the *ppc* gene may be obtained from NCBI's Genebank which is a known database, etc.

For example, the *ppc* gene of the present disclosure may be derived from microorganisms, but is not limited thereto.

The polynucleotide encoding the phosphoenolpyruvate carboxylase of the present disclosure may include a nucleotide sequence encoding an amino acid sequence described by SEQ ID NO: 50. As an example of the present disclosure, the polynucleotide of the present disclosure may have or may include the sequence of SEQ ID NO: 51. Further, the polynucleotide of the present disclosure may consist of, or may essentially consist of the sequence of SEQ ID NO: 51. Specifically, the phosphoenolpyruvate carboxylase may be encoded by the polynucleotide described by the nucleotide sequence of SEQ ID NO: 51.

In the polynucleotide of the present disclosure, various modifications may be made in the coding region as long as the amino acid sequence of the phosphoenolpyruvate carboxylase is not changed in consideration of codon degeneracy or codons preferred in organisms that are intended to express the phosphoenolpyruvate carboxylase of the present disclosure. Specifically, the polynucleotide of the present disclosure may include a nucleotide sequence having 70%, 75%, 80% 85%, 90%, 95%, 96%, 97%, 98%, 99%, 99.5%, 99.7% or 99.9% or more homology or identity to the sequence of SEQ ID NO: 51.

With respect to the objects of the present disclosure, the mutant polynucleotide including the nucleotide sequence encoding the phosphoenolpyruvate carboxylase of the present disclosure may regulate the content of 4-hydroxybutyrate (4HB) monomer of polyhydroxyalkanoate (PHA) produced therefrom, as compared to a polynucleotide including the nucleotide sequence encoding the phosphoenolpyruvate carboxylase and further including the nucleotide sequence encoding the wild-type 5'UTR.

For example, the mutant polynucleotide including the nucleotide sequence encoding the phosphoenolpyruvate carboxylase of the present disclosure may regulate the content of 4HB monomer of PHA produced therefrom in the range of 21.5% by weight to 46% by weight, based on the total weight of PHA. For example, with regard to the mutant polynucleotide including the nucleotide sequence encoding the phosphoenolpyruvate carboxylase of the present disclosure, the content of 4HB monomer of PHA produced therefrom may be about 21.5% by weight or more, specifically, 21.8% by weight or more, 22% by weight or more, 22.7% by weight or more, 23% by weight or more, 23.2% by weight or more, 24% by weight or more, 24.7% by weight or more, 25% by weight or more, 25.8% by weight or more, 26% by weight or more, 27% by weight or more, 28% by weight or more, 29% by weight or more, 29.1% by weight or more, 30% by weight or more, 31% by weight or more, 32% by weight or more, 33% by weight or more, 33.5% by weight or more, 34% by weight or more, 35% by weight or more, 36% by weight or more, 36.5% by weight or more, 37% by weight or more, 38% by weight or more, 39% by weight or more, 40% by weight or more, 40.3% by weight or more, 41% by weight or more, 41.2% by weight or more, 41.7% by weight or more, 42% by weight or more, 43% by weight or more, 44% by weight or more, 44.5% by weight or more, 45% by weight or more, 45.2% by weight or more, or 46% by weight or more, based on the total weight of PHA, but is not limited thereto.

As used herein, the term "polyhydroxyalkanoate (PHA)" is a polyester-based natural polymer accumulated in microorganisms, and is a biodegradable material, does not generate toxic waste, and is known as an eco-friendly polymer with thermoplastic and elastic properties, etc. Among PHA, the most representative known material is poly-3-hydroxybutyrate (P3HB) polymerized using 3-hydroxybutyrate (3HB) monomer. There is also a poly(3-hydroxybutyrate-co-4-hydroxybutyrate (P(3HB-co-4HB)) copolymer which is prepared by adding a 4-hydroxybutyrate (4HB) monomer to have characteristics as an elastomer, but is not limited thereto. In the present disclosure, the PHA may be specifically a PHA copolymer including a 4HB monomer while further including one or more monomers different from the 4HB, or while further including two, three, four, five, six or more monomers different from each other. More specifically, the monomer further included, together with the 4HB monomer, as the monomer different from the 4HB may be one or more monomers selected from the group consisting of 2-hydroxybutyrate, lactic acid, glycolic acid, 3-hydroxybutyrate (3HB), 3-hydroxypropionate (3HP), 3-hydroxyvalerate (3HV), 3-hydroxyhexanoate (3HH), 3-hydroxyheptanoate (3HHep), 3-hydroxyoctanoate (3HO), 3-hydroxyoctanoate (3HN), 3-hydroxydecanoate (3HD), 3-hydroxyundecanoate (3HDd), 4-hydroxyvalerate (4HV), 5-hydroxyvalerate (5HV), and 6-hydroxyhexanoate (6HH).

In particular, since physical properties of PHA vary depending on the addition ratio of 4HB monomer, which may be used in the production of various products, it may be industrially useful when the ratio of 4HB monomer may be adjusted.

Specifically, the degree of crystallization (crystallinity) of PHA may vary depending on the addition ratio of 4HB monomer, and more specifically, when the content of 4HB monomer increases, the crystallinity decreases, and PHA exhibits an amorphous property rather than a semi-crystalline property, which may lower processability. Therefore, it is important to maintain the processability of PHA by maintaining the appropriate content of 4HB monomer.

Accordingly, the mutant polynucleotide including the nucleotide sequence encoding phosphoenolpyruvate carboxylase of the present disclosure may produce PHA having the semi-crystalline property with excellent processability by regulating the content of 4HB monomer of PHA in the range of 21.5% by weight to 46% by weight, based on the total weight of PHA.

Still another aspect of the present disclosure provides a mutant polynucleotide encoding the mutant 5'UTR including the sequence in which any one or more bases selected from the group consisting of bases corresponding to positions 3, 4, 5, 7, 8, and 10 in the nucleotide sequence of SEQ ID NO: 1 are mutated; a mutant polynucleotide including a nucleotide sequence encoding phosphoenolpyruvate carboxylase, the mutant polynucleotide further including the nucleotide sequence encoding the mutant 5'UTR; or a vector including the same.

The mutant polynucleotide, the nucleotide sequence of SEQ ID NO: 1, and the mutation and mutant 5'UTR are as described above.

The vector may be an expression vector for expressing a target polynucleotide in a host cell, but is not limited thereto.

The vector of the present disclosure may include a DNA construct including the nucleotide sequence of the target polynucleotide operably linked to a suitable expression regulatory region (or expression regulatory sequence) so that the target polynucleotide, i.e., the mutant polynucleotide further including the nucleotide sequence encoding the mutant 5'UTR of the present disclosure may be expressed in a suitable host. The expression regulatory region may include a promoter capable of initiating transcription, any operator sequence for regulating the transcription, a sequence encoding a suitable RBS, and a sequence regulating termination of transcription and translation. The vector may be transformed into a suitable host cell, and then may replicate or function independently of the host genome, or may be integrated into the genome itself.

The vector used in the present disclosure is not particularly limited, but any vector known in the art may be used. Examples of commonly used vectors may include natural or recombinant plasmids, cosmids, viruses, and bacteriophages. For example, pWE15, M13, MBL3, MBL4, IXII, ASHII, APII, t10, t11, Charon4A, Charon21A, and the like may be used as a phage vector or a cosmid vector, and pDZ system, pBR system, pUC system, pBluescript II system, pGEM system, pTZ system, pCL system, pET system, and the like may be used as a plasmid vector. Specifically, pDZ, pDC, pDCM2, pACYC177, pACYC184, pCL, pECCG117, pUC19, pBR322, pMW118, pCC1BAC vectors and the like may be used.

For example, a target polynucleotide may be inserted into a chromosome through a vector for intracellular chromosome insertion. Insertion of the polynucleotide into the chromosome may be performed by any method known in the art, for example, homologous recombination, but is not limited thereto. The vector may further include a selection marker for the confirmation of chromosome insertion. The selection marker is for selecting the cells transformed with vectors, that is, for confirming the insertion of a target nucleic acid molecule, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector including a target polynucleotide is introduced into a host cell or a microorganism so that the polynucleotide may be expressed in the host cell. The transformed polynucleotide may be located by being inserted into the chromosome of the host cell or located outside the chromosome as long as it may be expressed in the host cell. The polynucleotide includes DNA and/or RNA encoding a target polypeptide. The polynucleotide may be introduced in any form as long as it may be introduced into a host cell and expressed. For example, the polynucleotide may be introduced into a host cell in the form of an expression cassette, which is a gene construct containing all elements required for autonomous expression. The expression cassette may usually include a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, the term "operably linked" means that the nucleotide sequence of the mutant polynucleotide further including the nucleotide sequence encoding the mutant 5'UTR of the present disclosure is functionally linked to a promoter sequence that initiates and mediates expression of the mutant polynucleotide further including the nucleotide sequence encoding the mutant 5'UTR.

In the present disclosure, the nucleotide sequence encoding the mutant 5'UTR, i.e., the mutant polynucleotide encoding the mutant 5'UTR may regulate translation of a polynucleotide encoding a target protein, which is operably linked thereto, into a protein. For example, the target protein may be phosphoenolpyruvate carboxylase, but is not limited thereto. It may be universally used for the purpose of regulating the translation of polynucleotides encoding various target proteins into proteins.

Still another aspect of the present disclosure may provide a polyhydroxyalkanoate-producing microorganism, the microorganism including the mutant polynucleotide encoding the mutant 5'UTR in which any one or more bases selected from the group consisting of bases corresponding to positions 3, 4, 5, 7, 8, and 10 in the nucleotide sequence of SEQ ID NO: 1 are mutated; or a mutant polynucleotide including a nucleotide sequence encoding phosphoenolpyruvate carboxylase, the mutant polynucleotide further including the nucleotide sequence encoding the mutant 5'UTR.

The mutation of the present disclosure may be any one or more selected from the following substitutions:
i) substitution of T with G;
ii) substitution of C with A;
iii) substitution of G with A; and
iv) substitution of G with C.

In one embodiment, the mutant 5'UTR of the present disclosure may include a polynucleotide in which T which is the base corresponding to position 3 in the nucleotide sequence of SEQ ID NO: 1 is substituted with G. The mutant 5'UTR of the present disclosure may include a polynucleotide in which C which is the base corresponding to position 4 in the nucleotide sequence of SEQ **ID** NO: 1 is substituted with A. The mutant 5'UTR of the present disclosure may include a polynucleotide in which T which is the base corresponding to position 5 in the nucleotide sequence of SEQ **ID** NO: 1 is substituted with G. The mutant 5'UTR of the present disclosure may include a polynucleotide in which G which is the base corresponding to position 7 in the nucleotide sequence of SEQ **ID** NO: 1 is substituted with A. The mutant 5'UTR of the present disclosure may include a polynucleotide in which G which is the base corresponding to position 8 in the nucleotide sequence of SEQ **ID** NO: 1 is substituted with C. Further, the mutant 5'UTR of the present disclosure may include a polynucleotide in which T which is the base corresponding to position 10 in the nucleotide sequence of SEQ ID NO: 1 is substituted with G. The mutant 5'UTR of the present disclosure may include a polynucleotide including any one or a combination of two or more, three or more, four or more, five or more, or six or more selected from the above-described substitutions.

The microorganism of the present disclosure may include any one or more selected from the group consisting of the mutant polynucleotide encoding the mutant 5'UTR including the sequence in which any one or more bases selected from the group consisting of bases corresponding to positions 3, 4, 5, 7, 8, and 10 in the nucleotide sequence of SEQ ID NO: 1 are mutated; the mutant polynucleotide including the nucleotide sequence encoding phosphoenolpyruvate carboxylase, the mutant polynucleotide further including the nucleotide sequence encoding the mutant 5'UTR; or a vector including the same.

As used herein, the term "microorganism (or strain)" includes all of wild-type microorganisms or naturally or artificially genetically modified microorganisms, and it may be a microorganism in which a specific mechanism is weakened or strengthened due to an insertion of a foreign gene or an activity enhancement or inactivation of an endogenous gene, and it may be a microorganism including a genetic modification for expression of a target polynucleotide. The microorganism may be a microorganism of the genus *Escherichia.* Specifically, the microorganism may be *Escherichia coli.*

The strain of the present disclosure may have a PHA-producing ability.

The strain of the present disclosure may be a recombinant strain prepared by introducing, into a natural wild-type microorganism, an unmodified microorganism, or a PHA-producing microorganism, a mutant nucleic acid molecule to be transcribed into the mutant 5'UTR of the present disclosure; or a mutant gene encoding phosphoenolpyruvate carboxylase including the mutant nucleic acid molecule to be transcribed into the mutant 5'UTR.

As used herein, the term "unmodified microorganism" does not exclude strains including mutations that may occur naturally in microorganisms, and may be a wild-type strain or a natural strain itself or may be a strain before the trait is changed by genetic variation due to natural or artificial factors. For example, the unmodified microorganism may be a strain into which the mutant polynucleotide encoding the mutant 5'UTR of the present disclosure; or the mutant polynucleotide including the nucleotide sequence encoding phosphoenolpyruvate carboxylase, the mutant polynucleotide further including the nucleotide sequence encoding the mutant 5'UTR is not introduced or has not yet been introduced. The term "unmodified microorganism" may be used interchangeably with "strain before being modified", "microorganism before being modified", "unvaried strain", "unmodified strain", "unvaried microorganism", or "reference microorganism".

With respect to the objects of the present disclosure, the strain of the present disclosure may regulate the content of 4HB monomer of polyhydroxyalkanoate produced therefrom in the range of 21.5% by weight to 46% by weight, based on the total weight of PHA, as compared to a strain expressing a gene encoding phosphoenolpyruvate carboxylase including a nucleic acid molecule to be transcribed into the wild-type 5'UTR.

For another example, in the recombinant microorganism of the present disclosure, the activity of some proteins in the PHA biosynthetic pathway may be additionally enhanced or weakened.

In the microorganism of the present disclosure, the mutant polynucleotide, the nucleotide sequence of SEQ ID NO: 1, the mutation, the mutant 5'UTR, and PHA are as described in other embodiments.

Still another aspect of the present disclosure provides a method of producing PHA, the method including the step of culturing, in a medium, a polyhydroxyalkanoate-producing microorganism, the microorganism including the mutant polynucleotide encoding the mutant 5'UTR in which any one or more bases selected from the group consisting of bases corresponding to positions 3, 4, 5, 7, 8, and 10 in the nucleotide sequence of SEQ ID NO: 1 are mutated; or the mutant polynucleotide including a nucleotide sequence encoding phosphoenolpyruvate carboxylase, the mutant polynucleotide further including the nucleotide sequence encoding the mutant 5'UTR.

The mutation of the present disclosure may be any one or more selected from the following substitutions:
i) substitution of T with G;
ii) substitution of C with A;
iii) substitution of G with A; and
iv) substitution of G with C.

In one embodiment, the mutant 5'UTR of the present disclosure may include a polynucleotide in which T which is the base corresponding to position 3 in the nucleotide sequence of SEQ ID NO: 1 is substituted with G. The mutant 5'UTR of the present disclosure may include a polynucleotide in which C which is the base corresponding to position 4 in the nucleotide sequence of SEQ ID NO: 1 is substituted with A. The mutant 5'UTR of the present disclosure may include a polynucleotide in which T which is the base corresponding to position 5 in the nucleotide sequence of SEQ ID NO: 1 is substituted with G. The mutant 5'UTR of the present disclosure may include a polynucleotide in which G which is the base corresponding to position 7 in the nucleotide sequence of SEQ ID NO: 1 is substituted with A. The mutant 5'UTR of the present disclosure may include a polynucleotide in which G which is the base corresponding to position 8 in the nucleotide sequence of SEQ ID NO: 1 is substituted with C. Further, the mutant 5'UTR of the present disclosure may include a polynucleotide in which T which is the base corresponding to position 10 in the nucleotide sequence of SEQ ID NO: 1 is substituted with G. The mutant 5'UTR of the present disclosure may include a polynucleotide including any one or a combination of two or more, three or more, four or more, five or more, or six or more selected from the above-described substitutions.

The method of producing PHA of the present disclosure may include the step of culturing, in a medium, a microorganism including any one or more selected from the mutant polynucleotide encoding the mutant 5'UTR including the sequence in which any one or more bases selected from the group consisting of bases corresponding to positions 3, 4, 5, 7, 8, and 10 in the nucleotide sequence of SEQ ID NO: 1 are mutated; the mutant polynucleotide including the nucleotide sequence encoding phosphoenolpyruvate carboxylase, the mutant polynucleotide further including the nucleotide sequence encoding the mutant 5'UTR; or a vector including the same.

As used herein, the term "culturing" means growing the microorganism of the present disclosure under appropriately controlled environmental conditions. The culture process of the present disclosure may be performed according to an appropriate medium and culture conditions known in the art. Such a culture process may be easily adjusted and used by those skilled in the art according to the selected microorganism. Specifically, the culturing may be a batch type, continuous type, and/or fed-batch type, but is not limited thereto.

As used herein, the term "medium" means a mixed substance containing nutrients, as main components, required to culture the microorganism of the present disclosure, and the medium supplies nutrients, growth factors, etc. including water, which are indispensable for survival and development. Specifically, as for the medium and other culture conditions used for culturing the microorganism of the present disclosure, any medium may be used without particular limitation as long as it is a medium commonly used for culturing microorganisms. The microorganism of the present disclosure may be cultured in a common medium containing proper carbon sources, nitrogen sources, phosphorus sources, inorganic compounds, amino acids and/or vitamins, etc., while controlling the temperature, pH, etc., under aerobic conditions.

In the present disclosure, the carbon sources include carbohydrates such as glucose, saccharose, lactose, fructose, sucrose, maltose, etc.; sugar alcohols such as mannitol, sorbitol, etc.; organic acids such as pyruvic acid, lactic acid, citric acid, etc.; amino acids such as glutamic acid, methionine, lysine, etc.; and the like. Natural organic nutrients such as starch hydrolysate, molasses, blackstrap molasses, rice bran, cassava, sugarcane residue, and corn steep liquor may be used. Specifically, carbohydrates such as glucose and sterilized pretreated molasses (namely, molasses converted to reducing sugar) may be used, and appropriate amounts of other carbon sources may be used in various manners without limitation. These carbon sources may be used alone or in a combination of two or more thereof, but are not limited thereto.

As the nitrogen sources, inorganic nitrogen sources such as ammonia, ammonium sulfate, ammonium chloride, ammonium acetate, ammonium phosphate, ammonium carbonate, ammonium nitrate, etc.; and organic nitrogen sources such as amino acids such as glutamic acid, methionine, and glutamine, etc., peptone, NZ-amine, meat extract, yeast extract, malt extract, corn steep liquor, casein hydrolysate, fish or decomposition products thereof, and skim soybean cake or decomposition products thereof, etc. may be used. These nitrogen sources may be used alone or in a combination of two or more thereof, but are not limited thereto.

The phosphorus sources may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, etc. may be used. In addition to these compounds, amino acids, vitamins and/or suitable precursors, etc. may be contained. These components or precursors may be added to the medium batchwise or continuously, but are not limited thereto.

During the culture of the microorganism of the present disclosure, pH of the medium may be adjusted by adding compounds such as ammonium hydroxide, potassium hydroxide, ammonia, phosphoric acid, and sulfuric acid to the medium in a proper manner. During the culture, foaming may be suppressed by using an antifoaming agent such as fatty acid polyglycol ester. Further, oxygen or oxygen-containing gas may be injected into the medium in order to maintain the aerobic state of the medium, or no gas may be injected or nitrogen, hydrogen, or carbon dioxide gas may be injected in order to maintain the anaerobic and microaerobic states, but is not limited thereto.

In the culture of the present disclosure, the culture temperature may be maintained at 20°C to 45°C, specifically, at 25°C to 40°C, and the microorganism may be cultured for about 10 hours to about 160 hours, but is not limited thereto.

PHA produced by the culture of the present disclosure may be secreted into the medium or may remain in the cells.

The method of producing PHA of the present disclosure may further include the step of preparing the microorganism of the present disclosure, the step of preparing a medium for culturing the microorganism, or a combination of these steps (in any order), for example, prior to the culturing step.

The method of producing PHA of the present disclosure may further include the step of recovering PHA from the medium according to the culture (the medium in which culturing is performed) or from the strain. The recovering step may be further included after the culturing step.

The recovering may be to collect the target PHA by way of a suitable method known in the art according to the method of culturing the microorganism of the present disclosure, for example, a batch, continuous, or fed-batch culture method. For example, centrifugation, filtration, treatment with a crystallized protein precipitant (salting out), extraction, sonication, ultrafiltration, dialysis, various forms of chromatography such as molecular sieve chromatography (gel filtration), adsorption chromatography, ion-exchange chromatography, affinity chromatography, etc., HPLC, or a combination thereof may be used. The target PHA may be recovered from the medium or microorganism by way of a suitable method known in the art.

The method of producing PHA of the present disclosure may further include the purifying step. The purifying may be performed by way of a suitable method known in the art. For example, when the method of producing PHA of the present disclosure includes both the recovering step and the purifying step, the recovering step and the purifying step may be performed continuously or discontinuously regardless of the order, or may be performed simultaneously or by being combined into one step, but is not limited thereto.

With respect to the objects of the present disclosure, the mutant gene encoding phosphoenolpyruvate carboxylase including the mutant nucleic acid molecule to be transcribed into the mutated 5'UTR, which is expressed by the strain of the present disclosure, may regulate the content of 4HB monomer of polyhydroxyalkanoate produced therefrom in the range of 21.5% by weight to 46% by weight, based on the total weight of PHA, as compared to a gene encoding phosphoenolpyruvate carboxylase including a nucleic acid molecule to be transcribed into the wild-type 5'UTR.

In the method of the present disclosure, the mutant polynucleotide, the nucleotide sequence of SEQ ID NO: 1, the mutation, the mutant 5'UTR, PHA, etc. are as described in the above other embodiments.

Still another aspect of the present disclosure provides a composition for producing PHA, the composition including a microorganism for producing PHA, the microorganism including the mutant polynucleotide encoding the mutant 5'UTR including the sequence in which any one or more bases selected from the group consisting of bases corresponding to positions 3, 4, 5, 7, 8, and 10 in a nucleotide sequence of SEQ ID NO: 1 are mutated; or the mutant polynucleotide including a nucleotide sequence encoding phosphoenolpyruvate carboxylase, the mutant polynucleotide further including the nucleotide sequence encoding the mutant 5'UTR; a medium in which the microorganism is cultured; or a combination of two or more thereof.

The composition of the present disclosure may further include arbitrary suitable excipients which are commonly used in compositions for producing PHA. Such excipients may be, for example, a preservative, a wetting agent, a dispersing agent, a suspending agent, a buffering agent, a stabilizer, or an isotonic agent, etc., but are not limited thereto.

Still another aspect of the present disclosure provides use of the microorganism in producing PHA, the microorganism including the mutant polynucleotide encoding the mutant 5'UTR including the sequence in which any one or more bases selected from the group consisting of bases corresponding to positions 3, 4, 5, 7, 8, and 10 in a nucleotide sequence of SEQ ID NO: 1 are mutated; or the mutant polynucleotide including a nucleotide sequence encoding phosphoenolpyruvate carboxylase, the mutant polynucleotide further including the nucleotide sequence encoding the mutant 5'UTR.

The mutant polynucleotide, the nucleotide sequence of SEQ ID NO: 1, the mutation, the mutant 5'UTR, PHA, etc. are as described in the above other embodiments.

### [Mode for Carrying Out the Invention]

Hereinafter, the present disclosure will be described in more detail with reference to exemplary embodiments. However, the following exemplary embodiments are only preferred embodiments for illustrating the present disclosure, and thus the scope of the present disclosure is not intended to be limited thereby. Meanwhile, technical matters not described in the present specification can be sufficiently understood and easily implemented by those skilled in the technical field of the present disclosure or similar technical fields. In addition, throughout the present specification, a number of papers and patent documents are referenced and citations thereof indicated. The entirety of the content disclosed in the cited papers and patent documents is incorporated in the present specification by reference in order to more clearly describe the level of the technical field to which the present disclosure belongs and the content of the present disclosure.

### Example 1: Construction of Library for Introducing Mutation into Ribosome-binding site (RBS) of ppc gene encoding Phosphoenolpyruvate Carboxylase and Screening

A template vector for the construction of an RBS mutant library in the 5'UTR sequence of *ppc* gene was prepared by the following method. The 5'-untranslated region (5'UTR) sequence of the wild-type *ppc* gene was amplified using the chromosome of *Escherichia coli* MG1655 as a template and a primer pair of SEQ ID NOS: 37 and 38. PCR conditions were repeated 25 cycles: denaturation at 95°C for 20 seconds, annealing at 56°C for 40 seconds, and extension at 72°C for 30 seconds. For fluorescence-based screening, a fluorescent protein to be expressed downstream of the promoter was amplified using a primer pair of SEQ ID NOS: 39 and 40. PCR conditions were repeated 25 cycles: denaturation at 95°C for 20 seconds, annealing at 56°C for 40 seconds, and extension at 72°C for 1 minute. The template vector was constructed by cloning the two amplified products and a pCL1920 vector treated with BamH1 restriction enzyme (NEB) in an assembly manner.

In order to prepare the RBS mutant library of *ppc* gene using the corresponding vector, a randomized sequence was amplified from the prepared template vector using a primer pair of SEQ ID NOS: 37 and 41. PCR conditions were repeated 25 cycles: denaturation at 95°C for 20 seconds, annealing at 56°C for 40 seconds, and extension at 72°C for 30 seconds. The remaining sequence of the template vector was amplified using a primer pair of SEQ ID NO: 42 and 43, and the amplified two fragments were ligated using an infusion cloning kit, and transformed into *E. coli* DH5α. PCR conditions were repeated 25 cycles: denaturation at 95°C for 20 seconds, annealing at 56°C for 40 seconds, and extension at 72°C for 3 minutes. For selection of the transformed strain, colonies were selected on an LB medium containing 75 mg/L of spectinomycin. For DNA amplification, AccuPower^{®} ProFi Taq PCR PreMix (Bioneer) was used.

Single colonies obtained by transforming the constructed library vector into E. *coli* MG1655 were seeded in a 96-well plate containing 500 µL of LB medium supplemented with 75 mg/L of spectinomycin, and incubated at 37°C for 16 hours. The saturated culture medium was diluted 1/100 with 500 µL of polyhydroxyalkanoate (PHA) production medium (US 10323261 B2), and fluorescence was measured after the cells were sufficiently saturated in the culture medium. At this time, a template vector having a 5'UTR sequence of the wild-type *ppc* gene was used as a control. Fluorescence and OD₆₀₀ were measured using a multilabel plate reader (PerkinElmer), and the measured fluorescence value was normalized by dividing by OD₆₀₀.

As a result, a total of 17 strains with different expression levels were identified, plasmids of the respective strains were isolated, each was named from Vector verB.1 to Vector verB.17, and sequenced to identify the mutant sequences.

The plasmids used above are shown in Table 1 below, and the primer sequences are shown in Table 2 below.

The mutant sequences of Vector verB.1 to Vector verB.17 identified above are shown in Table 3 below.

**[Table 1]**

| Plasmid name | Genotype |
|---|---|
| Template vector | pCL1920-Pppc-gfp, Sp^{R} |
| Library vector | pCL1920-Pppc*-gfp, Sp^{R} |
| Vector verB.1 | pCL1920-Pppc_vB1-gfp, Sp^{R} |
| Vector verB.2 | pCL1920-Pppc_vB2-gfp, Sp^{R} |
| Vector verB.3 | pCL1920-Pppc_vB3-gfp, Sp^{R} |
| Vector verB.4 | pCL1920-Pppc_vB4-gfp, Sp^{R} |
| Vector verB.5 | pCL1920-Pppc_vB5-gfp, Sp^{R} |
| Vector verB.6 | pCL1920-Pppc_vB6-gfp, Sp^{R} |
| Vector verB.7 | pCL1920-Pppc_vB7-gfp, Sp^{R} |
| Vector verB.8 | pCL1920-Pppc_vB8-gfp, Sp^{R} |
| Vector verB.9 | pCL1920-Pppc_vB9-gfp, Sp^{R} |
| Vector verB.10 | pCL1920-Pppc_vB10-gfp, Sp^{R} |
| Vector verB.11 | pCL1920-Pppc_vB11-gfp, Sp^{R} |
| Vector verB.12 | pCL1920-Pppc_vB12-gfp, Sp^{R} |
| Vector verB.13 | pCL1920-Pppc_vB13-gfp, Sp^{R} |
| Vector verB.14 | pCL1920-Pppc_vB14-gfp, Sp^{R} |
| Vector verB.15 | pCL1920-Pppc_vB15-gfp, Sp^{R} |
| Vector verB.16 | pCL1920-Pppc_vB16-gfp, Sp^{R} |
| Vector verB.17 | pCL1920-Pppc_vB17-gfp, Sp^{R} |

**[Table 2]**

| SEQ ID NO. | Sequence name | Sequence (5' -> 3') |
|---|---|---|
| 37 | primer 1 | CGACGGCCAGTGAATTCGAGCTCGGTACCCGGGGTCGGATGCGATACTT GCGCATC |
| 38 | primer 2 | CTCCAGTGAAAAGTTCTTCTCCTTTACTGACATTACTACGCAATGCGGAAT ATTG |
| 39 | primer 3 | GAACAATATTCCGCATTGCGTAGTAATGTCAGTAAAGGAGAAGAACTTTTC ACTG |
| 40 | primer 4 | GACCATGATTACGCCAAGCTTGCATGCCTGCAGGTCGACTCTAGAGCTCA AATGCCTGAGGTTTCAG |
| 41 | primer 5 | GCGGAATATTGTTCGTTCATATTNNNNNNNNNACCCCATC |
| 42 | primer 6 | ATGAACGAACAATATTCCGC |
| 43 | primer 7 | CCCGGGTACCGAGCTCGAATTCAC |

**[Table 3]**

| SEQ ID NO. | Name | 5'UTR sequence (5' -> 3') |
|---|---|---|
| 19 | Pppc (WT) | GATAAGATGGGGTGTCTGGGGTAAT |
| 20 | Pppc_vB1 | GATAAGATGGGGTGGAGGAGGGAAT |
| 21 | Pppc_vB2 | GATAAGATGGGGTGGAGGGGGTAAT |
| 22 | Pppc_vB3 | GATAAGATGGGGTGTAGGGGGTAAT |
| 23 | Pppc_vB4 | GATAAGATGGGGTGGATGGCGTAAT |
| 24 | Pppc_vB5 | GATAAGATGGGGTGGCGGAGGGAAT |
| 25 | Pppc_vB6 | GATAAGATGGGGTGTAGGAGGTAAT |
| 26 | Pppc_vB7 | GATAAGATGGGGTGTAGGACGTAAT |
| 27 | Pppc_vB8 | GATAAGATGGGGTGGATGACGTAAT |
| 28 | Pppc_vB9 | GATAAGATGGGGTGGATGAGGGAAT |
| 29 | Pppc_vB10 | GATAAGATGGGGTGTCTGGCGGAAT |
| 30 | Pppc_vB11 | GATAAGATGGGGTGGAGGAGGTAAT |
| 31 | Pppc_vB12 | GATAAGATGGGGTGGAGGACGTAAT |
| 32 | Pppc_vB13 | GATAAGATGGGGTGTCTGAGGTAAT |
| 33 | Pppc_vB14 | GATAAGATGGGGTGTCGGACGTAAT |
| 34 | Pppc_vB15 | GATAAGATGGGGTGGCTGAGGTAAT |
| 35 | Pppc_vB16 | GATAAGATGGGGTGTAGGAGGGAAT |
| 36 | Pppc_vB17 | GATAAGATGGGGTGTCGGAGGTAAT |

### Example 2: Construction of Mutant Sequence-Introduced Strain and Analysis of Monomer Content in Produced PHA

Based on a poly(3-hydroxybutyrate-co-4-hydroxybutyrate (P(3HB-co-4HB))-producing strain, a PHA-producing strain into which the mutant RBS sequence identified in Example 1 was introduced was prepared. As a parent strain, a P(3HB-co-4HB)-producing strain was used, in which the 4HB content in P(3HB-co-4HB) was about 20% (US 10323261 B2). First, mutant sequences of which sequences were identified were amplified using a primer pair of SEQ ID NOS: 44 and 45, respectively. The PCR conditions were repeated 25 cycles: denaturation at 95°C for 20 seconds, annealing at 56°C for 40 seconds, and extension at 72°C for 30 seconds. To perform gene recombination using a linear DNA fragment containing a selectable marker, an att-KanR-att DNA fragment was amplified using a primer pair of SEQ ID NOS: 46 and 47. PCR conditions were repeated 25 cycles: denaturation at 95°C for 20 seconds, annealing at 56°C for 40 seconds, and extension at 72°C for 2 minutes. In order to connect this fragment to the previously amplified sequence, overlap PCR was performed using a primer pair of SEQ ID NOS: 48 and 49. PCR conditions were repeated 25 cycles: denaturation at 95°C for 20 seconds, annealing at 56°C for 40 seconds, and extension at 72°C for 2 minutes and 15 seconds. After transforming the amplified DNA into the PHA-producing strain, strains into which the RBS mutation was introduced were selected in a selection medium containing 50 mg/L of kanamycin.

The primer sequences are shown in Table 4 below.

**[Table 4]**

| SEQ ID NO. | Sequence name | Sequence (5' -> 3') |
|---|---|---|
| 44 | primer 8 | CTTGATGGTTTCTCCCAGCACTTTGCCGAGCATACTGACATTACTACGCAATGCGGAA TATTG |
| 45 | primer 9 | CATAGCTGTGAAGCAGCTCCAGCCTACACTTAACATTTCCATAAGTTACG |
| 46 | primer 10 | CTTATGGAAATGTTAAGTGTAGGCTGGAGCTGCTTC |
| 47 | primer 11 | AATAATGTCGGATGCGATACTTGCGCATCTTATCCGACCTACACCTTTGGTGTTACTT GGGGCGATTTTATGGGAATTAGCCATGGTCC |
| 48 | primer 12 | CTTGATGGTTTCTCCCAGCAC |
| 49 | primer 13 | AATAATGTCGGATGCGATACTTG |

In order to evaluate the PHA-producing ability of 17 kinds of strains into which the RBS mutation was introduced, they were seeded in LB medium and cultured until sufficiently saturated. Thereafter, each strain was seeded in a 250 ml corner-baffle flask containing 25 ml of PHA production medium containing 5% glucose, and cultured with shaking at 230 rpm for 5 hours at 37°C and 43 hours at 35°C. After completion of the culture, the intracellular PHA content and the 4HB content in PHA were analyzed using gas chromatography. Specific culture conditions and analysis conditions were referred to the conditions previously used (US 10323261 B2).

The 4HB contents of the PHA-producing strains, into which the mutant sequence was introduced, are shown in Table 5 below.

**[Table 5]**

| Sequence name | PHA (mg) | 4HB content (wt%) |
|---|---|---|
| Pppc | 12.3 | 21.2% |
| Pppc_vB1 | 12.1 | 21.8% |
| Pppc_vB2 | 11.8 | 23.2% |
| Pppc_vB3 | 15.3 | 22.7% |
| Pppc_vB4 | 10 | 24.7% |
| Pppc_vB5 | 9.1 | 25.8% |
| Pppc_vB6 | 12.5 | 30.0% |
| Pppc_vB7 | 12.7 | 29.1% |
| Pppc_vB8 | 13.2 | 33.5% |
| Pppc_vB9 | 11.6 | 34.0% |
| Pppc_vB10 | 14.8 | 40.3% |
| Pppc_vB11 | 10.2 | 38.0% |
| Pppc_vB12 | 9.8 | 41.2% |
| Pppc_vB13 | 10.7 | 44.5% |
| Pppc_vB14 | 14.2 | 36.5% |
| Pppc_vB15 | 11.9 | 41.7% |
| Pppc_vB16 | 12 | 42.0% |
| Pppc_vB17 | 11.3 | 45.2% |

As shown in Table 4, the 4HB content in PHA was 21.2% in the control strain having the wild-type RBS sequence, whereas the 4HB content in PHA increased from 21.8% to 45.2% in the mutant strains having the mutant RBS sequence. Therefore, it was confirmed that the content of the PHA monomer may be effectively controlled by introducing the mutant RBS sequence into the PHA-producing strain.

Among 17 kinds of strains, into which the mutant RBS sequence was introduced, the Pppc_vB8 strain was named CB02-6607, and deposited at the Korean Culture Center of Microorganisms (KCCM), an international depository authority under the Budapest Treaty, on February 17, 2023, and assigned Accession No. KCCM 13332P.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the DEPOSITOR:
Escherichia coli CB02-6607 | Accession number given by the INTERNATIONAL DEPOSITARY AUTHORITY:
KCCM13332P

II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)

III. RECEIPT AND ACCEPTANCE
This International Depositary Authority accepts the microorganism identified under I above,
which was received by it on February. 17. 2023 (date of the original deposit).¹

IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: February. 17. 2023.

¹ Where Rule 6.4(d) applies, such date is the date on which the status of international depositary authority was
acquired.

Form BP/4 (sole page)
한국미생물보존센터 사단법인 한국종균협회
03641 서울특별시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea. Tel: 82-2-391-0950, 396-0950 Fax: 82-2-392-2859

## Claims

1. A mutant polynucleotide encoding a mutant 5'-untranslated region (5'UTR), comprising a sequence in which any one or more bases selected from the group consisting of bases corresponding to positions 3, 4, 5, 7, 8, and 10 in a nucleotide sequence of SEQ ID NO: 1 are mutated.

2. A mutant polynucleotide comprising a nucleotide sequence encoding phosphoenolpyruvate carboxylase, the mutant polynucleotide further comprising a nucleotide sequence encoding a mutant 5'UTR including a sequence in which any one or more bases selected from the group consisting of bases corresponding to positions 3, 4, 5, 7, 8, and 10 in a nucleotide sequence of SEQ ID NO: 1 are mutated.

3. The mutant polynucleotide of claim 1 or 2, wherein the mutation is any one or more selected from the following substitutions:
i) substitution of T with G;
ii) substitution of C with A;
iii) substitution of G with A; and
iv) substitution of G with C.

4. The mutant polynucleotide of claim 1 or 2, wherein the polynucleotide encoding the mutant 5'UTR includes any one or more nucleotide sequences selected from SEQ ID NOS: 2 to 18.

5. The mutant polynucleotide of claim 1 or 2, wherein the polynucleotide encoding the mutant 5'UTR includes any one or more nucleotide sequences selected from SEQ ID NOS: 20 to 36.

6. The mutant polynucleotide of claim 1, wherein the mutant polynucleotide encoding the mutant 5'UTR regulates translation of a polynucleotide encoding a target protein operably linked thereto into the protein.

7. The mutant polynucleotide of claim 6, wherein the target protein is phosphoenolpyruvate carboxylase.

8. The mutant polynucleotide of claim 2, wherein the mutant polynucleotide including the nucleotide sequence encoding phosphoenolpyruvate carboxylase regulates the content of a 4-hydroxybutyrate (4HB) monomer of polyhydroxyalkanoate produced therefrom in the range of 21.5% by weight to 46% by weight, based on the total weight of PHA, as compared to a polynucleotide including a nucleotide sequence encoding phosphoenolpyruvate carboxylase and further including a nucleotide sequence encoding the wild-type 5'UTR.

9. A polyhydroxyalkanoate-producing microorganism, the microorganism comprising a mutant polynucleotide encoding a mutant 5'UTR in which any one or more bases selected from the group consisting of bases corresponding to positions 3, 4, 5, 7, 8, and 10 in a nucleotide sequence of SEQ ID NO: 1 are mutated; or a mutant polynucleotide including a nucleotide sequence encoding phosphoenolpyruvate carboxylase, the mutant polynucleotide further including the nucleotide sequence encoding the mutant 5'UTR.

10. The microorganism of claim 9, wherein the content of 4HB monomer of polyhydroxyalkanoate produced from the microorganism is regulated in the range of 21.5% by weight to 46% by weight, based on the total weight of PHA.

11. The microorganism of claim 9, wherein the mutation is any one or more selected from the following substitutions:
i) substitution of T with G;
ii) substitution of C with A;
iii) substitution of G with A; and
iv) substitution of G with C.

12. A method of producing polyhydroxyalkanoate, the method comprising the step of culturing, in a medium, a polyhydroxyalkanoate-producing microorganism , the microorganism including a mutant polynucleotide encoding a mutant 5'UTR, in which any one or more bases selected from the group consisting of bases corresponding to positions 3, 4, 5, 7, 8, and 10 in a nucleotide sequence of SEQ ID NO: 1 are mutated; or a mutant polynucleotide including a nucleotide sequence encoding phosphoenolpyruvate carboxylase, the mutant polynucleotide further including the nucleotide sequence encoding the mutant 5'UTR.

13. The method of claim 12, wherein the mutant polynucleotide including the nucleotide sequence encoding phosphoenolpyruvate carboxylase regulates the content of 4HB content of polyhydroxyalkanoate produced therefrom in the range of 21.5% by weight to 46% by weight, based on the total weight of PHA, as compared to a polynucleotide including a nucleotide sequence encoding phosphoenolpyruvate carboxylase and further including a nucleotide sequence encoding the wild-type 5'UTR.

14. The method of claim 12, wherein the mutation is any one or more selected from the following substitutions:
i) substitution of T with G;
ii) substitution of C with A;
iii) substitution of G with A; and
iv) substitution of G with C.

15. Use of a mutant polynucleotide in producing polyhydroxyalkanoate, the mutant polynucleotide encoding a mutant 5'-untranslated region (5'UTR), in which any one or more bases selected from the group consisting of bases corresponding to positions 3, 4, 5, 7, 8, and 10 in a nucleotide sequence of SEQ ID NO: 1 are mutated.
